# EUROPEAN PATENT APPLICATION

(11) **EP 4 759 315 A1**
(43) Date of publication of application: **17.06.2026**
(21) Application number: 23948053.6
(22) Date of filing: 10.08.2023
(51) Int. Cl.: A61K 31/555, A61K 31/28, A61P 17/02

(54) **DRUG PREPARED FROM ORGANIC GERMANIUM COMPOUND AND GLUCOSAMINE COMPOUND, AND USE THEREOF**

(71) Applicant: Eion International Ltd., Taoyuan Taiwan 325 (TW)
(72) Inventor: HUANG, Chingtsuen, Taoyuan Taiwan 325 (CN)
(74) Representative: JWP Patent & Trademark Attorneys
(86) International application number: PCT/CN2023/112223
(87) International publication number: WO 2025/030478

(57) **Abstract**

A medicine prepared from an organogermanium compound and a glucosamine compound, and a use thereof, are characterized in that: (1) a high water-soluble salt medicine prepared from the organogermanium compound and the glucosamine compound, which is suitable for being prepared into various advantageous pharmaceutical dosage forms; and (2) the salt medicine retains the biochemical properties, pharmacological properties and medical efficacies of the components thereof. Moreover, because the pharmacological properties of the components thereof are medically complementary, the medicine has excellent effects in numerous medical uses including but not limited to wound healing.

## Description

### FIELD OF THE INVENTION

The present invention relates to a medicine prepared from an organogermanium compound and a glucosamine compound and use thereof, and in particular to a medicine that has analgesic, anti-inflammatory, antioxidant, antihypoxic, immunomodulatory, antiviral, antibacterial, coagulation-promoting, cell proliferation-accelerating, and other effects, is capable of stimulating the secretion of interferon and derivatizing transforming growth factors in organisms, as well as the medical use thereof in including but not limited to promoting the healing of various types of wounds.

### BACKGROUND OF THE INVENTION

Researches on the biochemical activity and medical applications of organogermanium compounds have spanned several decades; among them, some organogermanium compounds have been discovered to possess analgesic action, anti-inflammatory action, antioxidant effect, antihypoxic effect, immunomodulating action, and antiviral action (V. G. Lakhtin et al., "Synthesis and application of organogermanium compounds", Polymer Science Series D, Vol. 4, Iss. 3, pp 221-227, 2011-07-01; D. W. Niesel, C. B. Hess, Y. J. Cho, K. D. Klimpel and G. R. Klimpel, Infection and Immunity, June 1986, Vol. 52, No. 3, pp 828-833; L. G. Menchikovl and M. A. Ignatenko, "Biological Activity of Organogermanium Compounds - A Review", Pharmaceutical Chemistry Journal, Vol. 46, No. 11, February, 2013). Among them, the most widely and deeply studied are bis (2-carboxyethyl)germanium sesquioxide (Ge-132) of organogermanium sesquioxides and derivatives thereof. Ge-132 and its derivatives have been extensively researched and applied in anticancer, antihypertensive, liver disease prevention, anti-rheumatism (Pronai, L. and Arimori, S., "Decreased Plasma Superoxide Scavenging Activity in Immunological Disorder-Carboxyethylgermanium sesquioxide(Ge-132) as a Promoter of Prednisolone", Biotherapy, 1992, 4, 1-8), anti-cataract (Unaka, N.J. et al., 1995, "Effect of Germanium-132 on Galactose Cataracts and Glycation in Rats", Experimental Eye Research, 61, 155-164), osteoporosis inhibition, inflammation treatment, etc., and also have been used in the manufacture of cosmetics, nutritional supplements, and the like. Although the latest studies (Robin A. Reddeman et al., "A Toxicological Evaluation of Germanium Sesquioxide (Organic Germanium)", Hindawi, Journal of Toxicology, Vol. 2020, Article ID 6275625, 17 pages) have determined that the No-Observable-Adverse-Effect Level (NOAEL) of Ge-132 is 2000 mg/kg body weight/day, indicating it is a substance of extremely low toxicity, there exists a risk of severe nephrotoxicity caused by the contamination of germanium dioxide (GeO₂). Therefore, preventing the contamination of germanium dioxide has become a critical issue in the preparation of organogermanium pharmaceuticals. Preparing organogermanium in liquid form is an effective method to avoid contamination of solid germanium dioxide and to overcome the limitations of low water solubility in its applications, and is also one of the objectives of the present invention.

In addition to organogermanium sesquioxides and their derivatives, other types of organogermanium compounds, including germanes, germatranes, spirogermanium compounds, germanium porphyrines, and germanocenes, have also been disclosed to possess biological activities similar to those of bis(carboxyethyl)germanium sesquioxide (L. G. Menchikov and M. A. Ignatenko, "MOLECULAR-BIOLOGICAL PROBLEMS OF DRUG DESIGN AND MECHANISM OF DRUG ACTION: Biological Activity of Organogermanium Compounds (A Review)", Pharmaceutical Chemistry Journal, Vol. 46, No. 11, February, 2013).

Lots of studies have also demonstrated that organogermanium compounds can stimulate the secretion of γ-interferon and derive transforming growth factors (TGF) *in vivo,* which is considered an effective way for promoting wound healing. There are many causes of wounds; besides trauma, lacerations, abrasions, knife wounds, burns, scalds, sunburns, acne, diabetic ulcers, and pressure sores caused by long-term bed and other skin wounds, internal ulcers are also common wounds. Wound healing is a complex and slow process, with new epithelial and connective tissues formed through the migration and proliferation of cells during trauma treatment. Therefore, promoting cell migration, differentiation, proliferation, etc. contribute to wound healing. Previous studies (Paul Martin, "Wound Healing-Aiming for Perfect Skin Regeneration", p75-81, SCIENCE, Vol. 276, 4 April, 1997) have indicated that the so-called epidermal growth factor (EGF) family, including epidermal growth factors (EGFs), transforming growth factors-α (TGFs-α), and heparin-binding epidermal growth factors (HB-EGFs), will be abundantly released at epidermal injured parts and play a key role in regulating keratin at wound edges and in the formation of cell proliferation. Furthermore, studies have also found that when exogenous epidermal growth factors and TGF-α are applied to burn sites on pig backs, the regeneration of epidermal cells is enhanced (G. L. Brown et al., J. Exp. Med. 163, 1319 (1986); G. S. Schultz et al., Science 235, 350 (1987)). These growth factors act on the epidermis as motogens and mitogens, thus driving wound closure (Y. Barrandon and H. Green, Cell 50, 1131 (1987)).

Previous *in vitro* model studies on various cytokines and peptide growth factors affecting the recovery of intestinal epithelial cells have shown that four factors, i.e., TGF-α, epidermal growth factors, interleukin-1β (IL-1β), and interferon-γ (IFN-γ), promote the production of the bioactive TGF-β1 peptide with biological activity on wounded IEC-6 intestinal monolayer cell membranes and enhance the recovery of intestinal epithelial cell wounds by 2.3 to 5.5 times (Diagnass, A. U., Podolsky, D. K., Gastroenterology, 105(5), 1323-1332 (1993)).

In the prior art, growth factors are used as the main component of some wound healing agents. However, the treatment of wounds with such exogenous growth factors has the following drawbacks: (I) the growth factors are unstable proteins that degrade easily during storage and are digested and destroyed before entering into blood when administered orally; (II) the growth factors have a slow absorption rate in the human body and are rapidly degraded; hence, the efficacy of growth factor ointments for external application is limited; and (III) many growth factors are species-specific, for example, when administered parenterally, they may be recognized as foreign species to be rejected, causing dangerous immune reactions. For the above reasons, exogenous growth factors are not ideal wound-healing agents. Additionally, some literatures indicate that there is no evidence to show that exogenous growth factors administered parenterally will reach the skin, connective tissue, or supporting tissues. Therefore, only endogenous growth factors can truly and effectively promote wound healing. The major objective of the present invention is to prepare pharmaceuticals capable of producing endogenous growth factors.

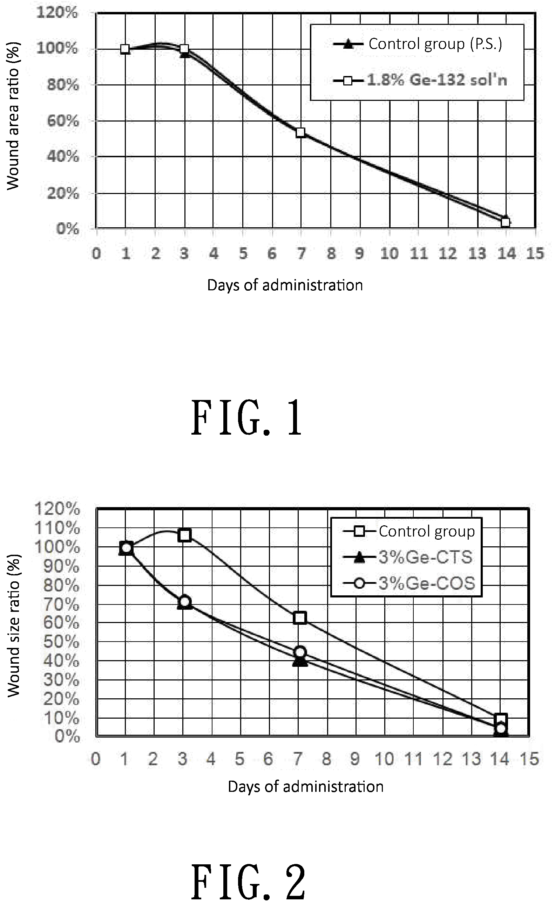

H. Matsumoto *et al.* conducted wound healing effect experiments in rats using an isotonic solution containing 1.8% Ge-132 (H. Matsumoto et al., "Restorative effect of organic germanium compound (Ge-132) on dermal injury", Wound Medicine 15, 6-10 (2016)). The experiments show that the effect in promoting wound contraction is not significantly different from that of normal saline. The experimental results are cited as Comparative Example 1 shown in FIG. 1. Although it demonstrated the effect in promoting wound contraction, there is no significant difference as compared to the saline control group, and the efficacy is inferior to that of the pharmaceuticals of the present invention. Developing wound healing pharmaceuticals with higher efficacy in promoting wound healing and capable of inhibiting exudate production is also one of the objectives of the present invention.

The other component for preparing the pharmaceuticals of the present invention is a glucosamine compound containing active amino groups, including glucosamine monosaccharides, disaccharides, polysaccharides of various degrees of polymerization (e.g., chitooligosaccharides and chitosan), and their derivatives. Glucosamine is a compound in which a hydroxyl group of glucose is replaced by an amino group, and is a natural amino monosaccharide. In the chitin of the shells of arthropods such as shrimps, and crabs, there are a large amount of glucosamine derivatives, N-acetylglucosamine. Therefore, polyglucosamine, commonly known as, chitosan or chito-NH₂ can be obtained by performing deacetylation on the chitin from shrimp and crab shells, with its molecular structure shown in Formula (I):

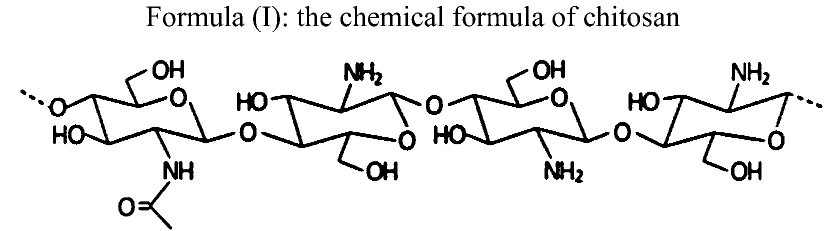

Generally, commercially available chitosan is not fully deacetylated, so as shown in Formula (I), there are varying amounts of N-acetyl amino groups depending on the degree of deacetylation.

Chitosan is a kind of polysaccharide that, through methods such as chemical hydrolysis, enzymatic degradation, or high energy impact, can be further depolymerized and concomitantly deacetylated to obtain chitooligosaccharides (COS-NH₂) with a lower degree of polymerization (DP) and higher degree of deacetylation. Chitooligosaccharides, besides having a lower degree of polymerization and higher degree of deacetylation, have a chemical structure similar to that of chitosan. For the sake of distinction, chitooligosaccharide is defined in some literature as polyglucosamine having a DP of ≤ 20 and an average molecular weight of ≤ 3900 Dalton (Da) (V. K. Mourya, N. N. Inamdar, and Y. M. Choudhari, "Chitooligosaccharides: Synthesis, Characterization and Applications", Polymer Science, Ser. A, 2011, Vol. 53, No. 7, pp. 583-612).

Glucosamine monosaccharides can also be obtained by the further depolymerization of chitosan or chitooligosaccharides, which is the major method for preparing glucosamine monosaccharides currently. Among them, enzymatic depolymerization is favored due to its advantage such as mild reaction conditions, high yield and specificity, and free of altering the structure of glucosamine (Pan et al., "Preparation of glucosamine by hydrolysis of chitosan with commercial α-amylase and glucoamylase", J. Zhejiang Univ-Sci B (Biomed & Biotechnol) 2011, 12(11):931-934). Glucosamine monosaccharide has a molecular weight of 179.17 and a degree of polymerization of 1, and it has similar properties to those of chitooligosaccharides in the preparation and application of the pharmaceuticals of the present invention.

Chitosan is a bioabsorbable polymer that has been widely used in food, materials, medical, agricultural, environmental protection, and other industries. Chitosan is highly biocompatible and non-toxic, and thus is used as a gene delivery vector in gene therapy. Further, due to its excellent adhesive properties, chitosan facilitates the transport of various agents across cell membranes and thus, is also used in controlled release of medicines for treating various diseases including cancer. Additionally, due to its physiological functions such as antibacterial, coagulation-promoting, cell proliferation acceleration, skin tissue repair promotion, tissue adhesion prevention, scar formation inhibition, and protection of joint cartilage, it is also used for hemostasis and wound healing.

Chitosan is insoluble in water, but for its applications, whether it is prepared to aqueous sol, hydrogel, solid microparticles, films, or fibers, etc. must be implemented in a way of dissolving. Therefore, to develop a preparation method of chitosan solution with simple procedure, short reaction time, without the addition of a solvent, and free from contamination of toxic components has always been a challenge in its medical applications. Solving this problem is also an objective of the present invention.

Different from chitosan, chitooligosaccharides have a higher degree of deacetylation, lower degree of polymerization, lower molecular weight and viscosity, and exhibit high water solubility. Moreover, chitooligosaccharides possess more remarkable pharmacological properties, such as antibacterial, antioxidant, anti-inflammatory, antihypertensive, and drug/DNA delivery functions. In addition, chitooligosaccharides also have demonstrated antidiabetic, anti-obesity, anti-HIV-1, anti-Alzheimer's disease, cholesterol-lowering, calcium absorption-enhancing, and hemostatic effects, as well as possessed superior cellular transduction and complete intestinal epithelial absorption as compared to chitosan. Therefore, chitooligosaccharides have been proven to be suitable for tissue engineering, agents or gene delivery, and overcoming multi-drug resistance in chemotherapy; their medical applications are as good as, and even greater than those of chitosan. So far, there are no findings of chitosan or chitooligosaccharides on inducing endogenous secretion of interferon or derivatizing growth factors. However, the existing studies have shown that chitosan can induce the release of platelet-derived growth factor-AB (PDGF-AB) and transforming growth factor-β1 (TGF-β1) from platelets (He Liu et al., "A functional chitosan-based hydrogel as a wound dressing and drug delivery system in the treatment of wound healing," RSC Adv., 2018, 8, 7533). The above research results concerning glucosamine compounds and organogermanium compounds have clearly demonstrated that the pharmacological properties of glucosamine compounds and organogermanium compounds are complementary to achieve a synergistic effect of medical efficacy such that the pharmaceuticals of the present invention has excellent efficacy.

### SUMMARY OF THE INVENTION

The present invention provides a medicine prepared from an organogermanium compound and a glucosamine compound, and use thereof. The present invention also has the following objectives, including: (I) preparing a liquid-form organogermanium medicine for convenient use and free of contamination of solid germanium dioxide; (II) developing a preparation method of chitosan solution with simple procedure, short reaction time, without the addition of a solvent, and free from contamination of toxic components; (III) preparing a medicine having both biochemical properties and medical effects of the organogermanium compound and the glucosamine compound; and (IV) preparing a medicine capable of generating endogenous TGFs in human and animal bodies *in vivo,* accelerating rapid wound healing and inhibiting the generation of wound exudate.

To achieve one or part or all of the above objectives or other objectives, in the present invention, the organogermanium compound containing carboxylic acid group (-COOH) is reacted with the glucosamine compound, and then a water-soluble salt medicine is prepared by means of protonation of the reactive amino group (-NH₂) of the glucosamine compound by the hydrogen ion on the carboxylic acid group of the organogermanium compound. This salt medicine possess high water solubility, can be dissociated in tissue fluid, and exhibit the biochemical properties, pharmacological characteristics, and medical effects of components thereof. These biochemical properties, pharmacological characteristics, and medical effects include: analgesic, anti-inflammatory, antioxidant, antihypoxic, immunomodulating, and antiviral effects, induction of interferon secretion, and derivation of TGFs derived from the organogermanium compound component; and antibacterial, antioxidant, anti-inflammatory, antihypertensive, drug/DNA delivery, antidiabetic, anti-obesity, anti-HIV-1, anti-Alzheimer's disease, cholesterol-lowering, calcium absorption enhancement, hemostatic, cell transduction effects, and complete absorption through the intestinal epithelium, derived from the glucosamine compound component. The complementary effects described above endow the medicine of the present invention have outstanding medical efficacy in multiple aspects, including but not limited to promoting the healing of wounds (including trauma, lacerations, abrasions, knife wounds, burns, scalds, sunburns, acne, diabetic ulcers, and pressure sores caused by long-term bed, etc.). The following provides examples of preparing the medicine of the present invention using Ge-132 and chitosan and chitooligosaccharides, as well as its use in accelerating wound healing, to demonstrate the excellent medical efficacy of the medicine.

The above description is merely an overview of the technical solution of the present invention. To understand the technical means of the present invention more clearly and to implement the present invention according to the disclosure of the description, and to make the above and other objectives, features, and advantages of the present invention more obvious and understandable, preferred embodiments are specifically enumerated below in combination with the accompanying drawings, as detailed below.

### BRIEF DESCRIPTION OF THE DRAWINGS

FIG. 1 shows temporal changes in a wound size ratio of rats in Comparative Example 1.
FIG. 2 shows temporal changes in a wound size ratio of rabbits in Example 1.
FIG. 3 shows temporal changes in a wound size ratio of rabbits in Example 2.
FIG. 4 shows temporal changes in a wound size ratio of rabbits in Example 3.

### DETAILED DESCRIPTION OF PREFERRED EMBODIMENTS

The organogermanium compound carrying carboxylic acid group suitable for preparing the medicine of the present invention includes, but is not limited to, organogermanium sesquioxides, germanes, germatranes, spirogermanium compounds, germanium porphyrines, and germanocenes, among which the most typical is bis-carboxyalkyl germanium sesquioxide. The chemical formula of its monomer is shown in Formula (II):

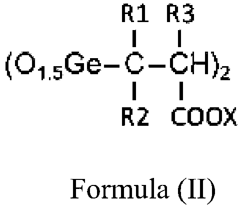

In the formula, R1, R2, and R3 may be H, substituted or unsubstituted low-carbon alkyl (such as methyl or ethyl), aryl, hetaryl, substituted or unsubstituted amino group or amido group, etc.; X may be H, K, Na, basic amino group, aryl, or hetaryl, etc. When R1, R2, R3, and X in Formula (II) are H, it is bis(carboxyethyl)germanium sesquioxide, a solid polymer with the molecular formula of n(GeO_{1.5}CH₂CH₂COOH)₂, abbreviated as Ge-132. When X in Formula (II) is K, Na, basic amino group, aryl, or hetaryl, etc., white Ge-132 powder may be obtained by the acidification and crystallization using concentrated hydrochloric acid. In addition, the chemical formula of germatranes is shown in Formula (IV):

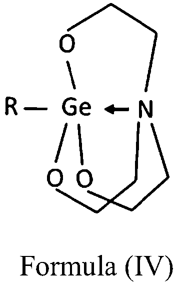

In the formula, R is one of cinnamic acid group [-CH(C₆H₄OH)CH₂COOH], caffeic acid group [-CH(C₆H₃(OH)₂)CH₂COOH], or derivatives thereof. The spirogermanium compound is selected from at least one of the following: 3-(2,8,9-trioxa-aza-1-germatricyclo-[3,3,3,0]-undecane-1-yl) caffeic acid, 3-(2,8,9-trioxa-aza-1-germatricyclo-[3,3,3,0]-undecane-1-yl)-10-hydroxy-decanoic acid, 3-(2,8,9-trioxa-aza-1-germatricyclo-[3,3,3,0]-undecane-1-yl)-10-methoxy-decanoic acid, 3-(2,8,9-trioxa-aza-1-germatricyclo-[3,3,3,0]-undecane-1-yl)-10-ethoxy-decanoic acid, and 3-(2,8,9-trioxa-aza-1-germatricyclo-[3,3,3,0]-undecane-1-yl)-10-phenyl-decanoic acid.

Ge-132 is the most representative bis-carboxyalkyl germanium sesquioxide, and its structural formula is shown in Formula (III):

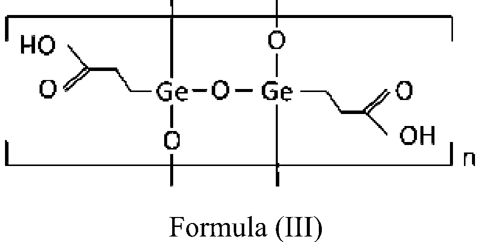

Ge-132 is soluble in water. The reaction of dissolution into water is shown in Equation (1):

n(GeO_{1.5}CH₂CH₂COOH)₂+ 3nH₂O = 2nGe(OH)₃CH₂CH₂COOH (1)

When Ge-132 is dissolved into water, a carboxylic acid called 3-(trihydroxygermyl)propanoic acid (THGP) is generated, with the chemical molecular formula of Ge(OH)₃CH₂CH₂COOH.

### Description of the principle of medicine preparation

Chitosan is one of the glucosamine compounds used in the present invention, and it is set as an example for specification below. As shown in Formula (I), the chemical structure of chitosan contains three common reactive functional groups: i.e., amino group, primary hydroxyl group, and secondary hydroxyl group. Therefore, it has higher reactivity than general polysaccharides, but is insoluble in water. In various medical applications of chitosan, it is mainly made into sols, gels, films, fibers, or other forms; liquefaction is the first issue encountered in the preparation of these dosage forms. Previous studies have revealed as follows: chitosan is only soluble in acidic solutions, and the dissolution of chitosan attributes to the protonation of its reactive amino group by hydrogen ions. The solubility will be affected by its degree of deacetylation, the distribution of acetyl groups on the molecular chain, the pH of the acidic solution, the structure of the acid molecule, and other ions present. Therefore, studies have found that the highest pH value at which chitosan may be dissolved ranges from pH 6 to pH 6.5 (Domard A. "pH and c.d. Measurements on a Fully Deacetylated Chitosan: Application to Cu II-polymer Interactions" Int. J. Biol. Macromol., 9, 98, 1987; J. W. Park and H. K. Choi, "Acid-Base Equilibria and Related Properties of Chitosan" Bull. Korean Chem. Soc., Vol. 4, No. 8, 1983; Rinaudo, M.; Pavlov, G.; Desbrières, J. "Solubilization of Chitosan in Strong Acid Medium" Int. J. Polym. Anal. Charact. 5, 267-276, 1999). Among them, Rinaudo *et al.* further indicated that when more than 50% of the reactive amino groups in chitosan molecules are protonated, chitosan is in a dissolved state.

The carboxylic acid group THGP formed by the dissolution of Ge-132 in water is a weak acid. According to the tests of Sara M. Ogwapit *et al.* (Sara M. Ogwapit et al., "Analysis of Ge-132 and development of a simple oral anticancer formulation", Bioscience Horizons, vol. 4, No. 2, June 2011), the solubility of Ge-132 in water at 25°C is 1.28 g/100 ml, and the pH of the solution is 3.06-3.12 at this concentration. Experiments of the present invention show that THGP solution, like acetic acid solution, has good dissolving effect on chitosan. This dissolving effect attributes to the protonation of the reactive amino group (-NH₂) on chitosan by the hydrogen ions on the carboxylic acid group of THGP, and the reaction is shown in Equation (2).

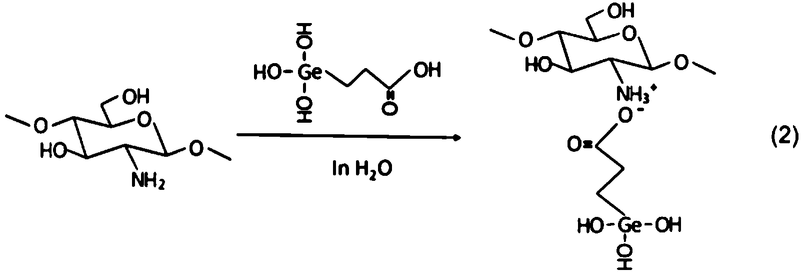

The reaction product in Equation (2) is chitosan-N-3-trihydroxygermyl propionate (chito-N-THGP) which is a polycationic polymer-type water-soluble salt compound. In water, chito-N-THGP is dissociated into 3-(trihydroxygermyl) propionate anions (Ge(OH)₃CH₂CH₂COO-) and protonated chitosan cations (chito-NH₃⁺), thus exhibiting the biochemical properties of these ions.

Experiments of the present invention show that the reaction in Equation (2) is a mild process. When chitosan is added to a solution of 3-(trihydroxygermyl)propanoic acid, chitosan is protonated to produce a pale orange-yellow, viscous reaction solution containing chito-N-THGP, which is a colloidal solution. As the amount of chitosan added increases, the pH and viscosity of the reaction solution also increase. According to the experiments of the present invention, when the pharmaceutical is prepared using chitosan having a degree of deacetylation of 91.35% and a molecular weight of 100-130 kDa, and the dissolved chitosan reaches 5% of the total weight of the reaction solution, the viscosity (at 25°C) will rise to about 15,000 cp. When the amount of chitosan added reaches a certain level, the hydrogen ions of the carboxylic acid group of THGP are used up, and the reaction in Equation (2) is ended, chitosan is no longer dissolved, and the reaction solution becomes turbid instead of clear and transparent. At this point, the pH of the reaction solution is about 6.

Due to the variability in the degree of polymerization, degree of deacetylation, and molecular weight of chitosan, as well as their impacts on the solubility, it is difficult to control the quality of the medicine. Therefore, the present invention specifically adopts acids with good dissolving ability for chitosan, including but not limited to acetic acid, formic acid, hydrochloric acid, sulfuric acid, or other acidic agents, as a pH regulator, such that the dissolution of chitosan no longer relies solely on THGP. In this way, each functional raw material may be dissolved according to the predetermined demand ratio, moreover, the medicine may be ensured to have stable appearance and properties. Besides being ideal pH regulators, these acidic agents may also substitute for the function of THGP, such that the amount of chitosan and THGP used in medicine preparation may be freely adjusted and not limited to the reaction equivalents. Therefore, pH regulators may not only facilitate the preparation of pH-stable, transparent, and clear medicine solutions, but also may serve as viscosity modifiers. That is, the required viscosity may be achieved without the need for additional thickening agents, thus ensuring the purity of the medicine components and reducing the risk of contamination. Similarly, the addition of alkaline pH regulators, including but not limited to, ammonium hydroxide or hydroxides of alkali and alkaline-earth metals, such as sodium hydroxide, potassium hydroxide, magnesium hydroxide, and calcium hydroxide, may promote the dissolution of organogermanium compounds carrying carboxylic acid groups, such that their content in the pharmaceutical solution is no longer limited by the reaction equivalents with chitosan. Thus, in addition to pH adjustment, the medicine of the present invention may be allowed to further contain highly water-soluble salts formed by these alkaline agents and organogermanium compounds. This is beneficial for increasing the concentration of 3-(trihydroxygermyl)propionate anion (Ge(OH)₃CH₂CH₂COO-) and the corresponding medical efficacy, which is also part of the present invention. According to the research of the present invention, the salts formed by the aforesaid alkaline agents and Ge-132 all exhibit excellent effects in promoting wound healing. Further, calcium ions additionally possess a hemostatic effect. Therefore, when calcium hydroxide is used as the alkaline agent, the prepared medicine is also advantageous for wound hemostasis, which will be demonstrated in the examples.

The above reaction solution containing chito-N-THGP, prepared from chitosan, exhibits typical properties similar to those of other organic acid solutions of chitosan, including: (1) the reaction solution is in a sol state (i.e., sol solution) under acidic conditions and transforms into a gel state under neutral or alkaline conditions; (2) the salt compound (chito-N-THGP) formed by the reaction is insoluble in organic solvents, including alcohol. Therefore, besides being prepared into forms such as ointments or liquid formulations, the sol solution is prepared into other forms for use by means of these properties. For example: the sol solution is adjusted to neutral or alkaline conditions, subjected to gelation and then prepared as a hydrogel; dispersed into 95% alcohol or organic solvents to obtain a precipitate; then the precipitate is filtered and vacuum-dried at low temperature to produce a powder. The sol solution may be subjected to electrospinning alone or blended with other suitable high-molecular compounds such as but not limited to polyvinyl alcohol (PVA), by the electrospinning technology, to produce fibers, fiber membranes (El-Refaie Kenawy et al., Journal of Controlled Release 81(2002) 57-64; Zheng-Ming Huang et al., Composites Science and Technology 63 (2003) 2223-2253; Y. Liu et al., J. Biomed. Mater. Res. Part B: Appl Biomater. 90B:492-502, 2009), or nonwoven fabrics. Additionally, experiments of the present invention also show that the sol solution has good miscibility with glycerol. The sol solution is thoroughly mixed with an appropriate amount of glycerol (boiling point: 290°C), then coated as a thin layer and heated up appropriately to remove moisture, a soft and transparent film may be then produced. Therefore, the medicine of the present invention, besides being prepared as liquids, ointments, and emulsions, may also be prepared as forms such as powders, films, fibers, fiber membranes, or nonwoven fabrics. The medicament of the present invention may also be added with at least one auxiliary agent as needed and is suitable for *in vivo* or *in vitro* administration.

In addition to chitosan, the present invention also utilizes chitooligosaccharide and THGP for pharmaceutical preparation. The experimental results of the present invention using chitooligosaccharide with an average molecular weight of 1270 Da, obtained by enzymatic degradation, show that the aqueous solution of chitooligosaccharide is faintly acidic, with a pH of 5.4 for a 1% solution at 25°C. Concentration has little effect on pH; when the concentration increases from 1% to 10%, the pH does not change significantly. However, temperature variation has a significant effect on pH. As temperature increases, the pH decreases. For example, 10% chitooligosaccharide solution is set as an example, when temperature rises from 20°C to 55°C, the pH decreases from 5.65 to 4.49, showing an almost linear trend.

The protonation reaction between chitooligosaccharide and THGP may also be represented by Equation (2). The reaction product is COS-N-3-trihydroxygermyl propionate, (COS-N-THGP), which is also a salt compound. In water, COS-N-THGP will be dissociated into 3-(trihydroxygermyl)propionate anions (Ge(OH)₃CH₂CH₂COO-) and protonated chitooligosaccharide (COS-NH₃⁺) cations, and thus, possesses the biochemical properties of both the components.

For the preparation of a medicament with chitooligosaccharide, its operation is the same as that with chitosan. When chitooligosaccharide is added to a THGP solution, it is dissolved to form a reaction solution having brown color. Due to the high water solubility of chitooligosaccharide, its dissolution is not completely controlled by the protonation reaction of amino groups, and the pH of the reaction solution is also much lower than that prepared with chitosan. Because the protonation reaction with chitooligosaccharide consumes more Ge-132 to promote the dissolution of Ge-132, like the dissolution of chitosan, the concentration of 3-(trihydroxygermyl)propionate in the reaction solution may also exceed its saturation concentration in the Ge-132 aqueous solution. Since chitooligosaccharide has both low degree of polymerization and low molecular weight, the viscosity of the reaction solution is also low, so there is no operation issue caused by high viscosity, nor is there a need to lower the pH to aid the dissolution of chitooligosaccharide. Conversely, alkali solutions, including but not limited to, ammonia water or hydroxides of alkali and alkaline-earth metals such as sodium hydroxide and calcium hydroxide, may be added as needed, to moderately increase the pH or, if necessary, to facilitate the dissolution of Ge-132, thereby increasing the concentration of 3-(trihydroxygermyl)propionate in the pharmaceuicals as previously described. When calcium hydroxide is used as the pH regulator, the calcium ions have a hemostatic effect, it may be also beneficial to wound hemostasis. The medicine of the present invention prepared with chitooligosaccharide may be also formulated into liquids, ointments, and powders by adding suitable excipients and additives; and also, may be made into films by adding chitosan gel solution. However, due to its high hygroscopicity, moisture-proof treatment needs to be considered when making powders, avoiding caking.

Based on the above description, the preparation steps of the medicine of the present invention are summarized as follows: (1) Material preparation: according to the desired quantity and components of the medicine, preparing the required raw materials including organogermanium compounds such as Ge-132, glucosamine compounds, e.g., chitosan or chitooligosaccharide, pH regulators, and deionized water; (2) Charging water and stirring with optional heating : placing the required amount of deionized water in a heatable stirring reaction vessel, stirring, and heating as needed; (3) Addition of the organogermanium compound: adding the required amount of organogermanium compound by dry weight to the reaction vessel, dispersing and dissolving in water under stirring conditions to generate THGP; (4) Addition of the glucosamine compound: when the water temperature reaches a predetermined temperature, adding the glucosamine compound to the reaction vessel, and avoiding the poor dispersion of solid particles caused by excessive speed; (5) Continuous stirring to complete the protonation reaction: after the glucosamine compound is fully added, stopping heating at an appropriate time (ifheated), continuing monitoring until the pH of the reaction mixture is stable, to confirm the completion of the protonation reaction; (6) pH adjustment: adjusting the pH of the reaction mixture to a predetermined value using a pH regulator; and (7) Weight adjustment: deionized water is added to adjust to a predetermined value, thereby completing the preparation.

The above steps are simple and readily implementable. Matters related to the operation efficiency and medicine quality control are as follows: In step (1), since chitosan and chitooligosaccharide are hygroscopic, be sure to pay attention to moisture-proof storage, and the water content should be determined first before preparation; their reaction equivalents may vary with the molecular structure; hence, batch analysis and determination are required. In step (2), the amount of water placed into the reaction vessel should be selected so as to allow sufficient capacity for the subsequent addition of the pH regulator solution (although the amount used is small). Heating may or may not be applied. Heating may promote the dissolution of Ge-132 and may also accelerate the reaction, but subsequent cooling is more time-consuming; if room temperature is below 25°C, heating is preferable, but the temperature should not exceed 50°C. In step (3), the organogermanium compound may be added all at once or in portions, and may be added before heating the water or when the heating is completed. In step (4), according to the predetermined pharmaceutical composition, chitosan or chitooligosaccharide is added in batches. When preparing pharmaceuticals with chitosan, the viscosity of the reaction solution will increase with the addition of chitosan, and the flow pattern of the reaction solution may change during stirring, so excessive feeding should be avoided to prevent the poor dispersion of particles, and a stirrer suitable for high-viscosity fluids should be used. When preparing pharmaceuticals with chitooligosaccharide, due to its high water solubility and low viscosity of the reaction solution, there exists no operation issue caused by viscosity. In step (5), when a stable pH is measured, it indicates the completion of the protonation reaction and allows proceeding to the subsequent steps. Since the pH of the reaction solution will be affected by temperature, temperature correction needs to be performed during measurement. In step (6), during pH adjustment, temperature correction is required. Moreover, pay attention to the potential damage to the pH electrode caused by the viscosity of the medical solution, and to avoid affecting the accuracy of the measurement. In step (7), component adjustment should be based on weight to avoid the influence of temperature variation, and may be performed without waiting for cooling.

The above pharmaceuticals are first disclosed by the present invention. To make the features, objectives, and advantages of the present invention more obvious and understandable, the following provides a detailed description of the use of the pharmaceuticals in promoting wound healing as an example, with reference to the accompanying drawings. This example should not be construed as limiting the types of the pharmaceuticals or other medical uses of the present invention.

Comparative Example 1 on the effect of wound healing: literature discloses that Hiroko Matsumoto *et al.* used Ge-132 solution, i.e., THGP solution, to study skin wound healing in rats (Hiroko Matsumoto et al., "Restorative effect of organic germanium compound (Ge-132) on dermal injury", Wound Medicine, Vol.15, Dec. 2016, P6-10). The surgical wound of rats is treated with 1.8% Ge-132 isotonic solution, and physiological saline solution (PS) treatment is as a control for comparison. The study found that the wound area of the administered group is 1.45 ± 0.23 cm² on day 1, 1.45 ± 0.13 cm² on day 3, 0.780 ± 0.124 cm² on day 7, and 0.050 ± 0.013 cm² on day 14, respectively. In the control group administered with physiological saline solution (P. S.), the wound area is 1.77 ± 0.36 cm², 1.73 ± 0.13 cm², 0.942 ± 0.150 cm², and 0.108 ± 0.049 cm², respectively. For the sake of clear comparison, based on the above results, the wound area ratios on days 1, 3, 7, and 14 are compared by setting the median wound area on day 1 as 100%, as shown in Fig. 1. The two curves represent the wound area ratio changes in the administered and control groups during the administration period are very similar, indicating that the effect of promoting wound healing of the Ge-132 isotonic solution is not particularly significant.

Example 1: Example 1 involves the use of the medicine of the present invention for wound healing treatment to demonstrate inventive step of the present invention. The medicine used includes: pharmaceuticals containing (1) Ge-132 equivalent of 3 wt% and chitosan equivalent of 4.5% (abbr.: 3% Ge-CTS), (2) Ge-132 equivalent of 3% and chitooligosaccharide equivalent of 4.5% (abbr.: 3% Ge-COS), (3) Ge-132 equivalent of 1.5% and chitosan equivalent of 2.25% (abbr.: 1.5% Ge-CTS), which is obtained by diluting 3% Ge-CTS with deionized water. For comparison with Comparative Example 1, the organogermanium component in the above experimental pharmaceticals is measured by the Ge-132 equivalent contained therein. The so-called Ge-132 equivalent of 3% means that the weight of Ge-132 used in medicine preparation is 3% of the total weight of the resulting medicine. According to the molecular weight (339.4222) of Ge-132, the molecular weight (199.75) of THGP, and the reaction equivalent relationship in Equation (1), the Ge-132 content of 3% is equivalent to THGP content of 3.531%, or to germanium element (atomic weight: 72.64) content of 1.284%.

These examples are intended to demonstrate the wound healing effect of the present invention and should not be construed as limiting the scope of rights of the present invention. In addition, to understand and compare the effects of different Ge-132 salts on wound healing, a calcium 3-(trihydroxygermyl)propionate solution containing Ge-132 equivalent of 3% (abbr.: 3% Ge-Ca) is also adopted in the present invention for wound healing experiments, which is presented as Example 2 to examine the wound healing effect of Ge-132 alkaline-earth metal salts.

### Preparation of experimental pharmaceuticals in the examples:

The preparation is performed according to the aforementioned methods and steps.

### Material preparation:

The organogermanium compound used is Ge-132. Commercially available fine Ge-132 powder having a purity of over 99.95% is taken and dissolved in deionized water at 95°C first to form a saturated aqueous solution, then the saturated aqueous solution is filtered with Whatman No. 3 qualitative filter paper. The filtrate is collected in a beaker, stirred, and cooled to room temperature to crystallize Ge-132. The crystalline slurry is then vacuum filtered, and the filter cake is washed with deionized water and dried to a water content of 0.15%, and finally, crushed into powder for later use. The glucosamine compounds are chitosan and chitooligosaccharide, both are commercially available products (Cheng Li Industrial Co.). The chitosan has the degree of deacetylation of 91.35%, the molecular weight of 100-130 kDa and a moisture content of 9.47 wt%; the chitooligosaccharide has the degree of deacetylation of 91.35%, the average molecular weight of 1270 Da and a moisture content of 8.32 wt%. The pH regulator used is a 10 wt% aqueous solution of glacial acetic acid. Since chitosan is hygroscopic, it is dried to remove water before use, and the dry weight is measured for use.

### Preparation of the experimental pharmaceutical 3% Ge-CTS:

Preparation according to the following steps: (1) 180 g of deionized water is taken into a 500 ml beaker, with the addition of 6 g (dry weight) of Ge-132 powder, and electric stirring is started, as well as heating up to 50°C; (2) 9 g (dry weight) of chitosan (i.e., 1.5 times the weight of Ge-132) is taken, and slowly added to the stirring Ge-132 solution; (3) after the addition of chitosan is completed, stirring is continued and the temperature is maintained for 30 minutes, then heating is stopped and the temperature is allowed to drop to 25°C, and the pH of the reaction solution is measured 6.03; (4) the pH of the reaction solution is adjusted to 5.81 using a pH regulator; (5) deionized water is added to adjust the weight to 200 g, and the solution is mixed well to obtain a pale orange-yellow viscous liquid. The 9 g of chitosan used in the above preparation (1.5 times the weight of Ge-132) is determined by experiments in advance as the weight required for the protonation reaction with 6 g of Ge-132.

### Preparation of the experimental pharmaceutical 3% Ge-COS:

The preparation is performed using the same procedure and temperature as for the preparation of 3% Ge-CTS, using the same Ge-132, but without the use of a pH regulator. The prepared COS-3-trihydroxygermyl propionate is a low-viscosity brown solution, containing a Ge-132 equivalent of 3 wt% and a chitooligosaccharide equivalent of 4.5 wt%, with a measured pH of 3.02 at 25°C. For the convenience of comparison with chitosan above, the weight of chitooligosaccharide used is thus also 1.5 times the weight of Ge-132.

### Preparation of the experimental pharmaceutical 3% Ge-Ca:

(1) 180 g of water is taken and placed in a 500 ml beaker and stirred with an electromagnetic heating stirrer, then 6.01 g of Ge-132 powder (dry weight of 6 g) is taken into the water. (2) Separately, 1.31 g of Merck GR-grade Ca(OH)₂ powder is taken and added to the beaker, heated to 45°C to dissolve the Ge-132 powder and Ca(OH)₂ powder. 30 minutes later, water is added to adjust the total solution weight to 200 g, then the solution is filtered through Whatman No. 3 qualitative filter paper to obtain the experimental pharmaceutical of 3% Ge-Ca containing Ge-132 equivalent of 3 wt%, with a measured pH of 7.04 at 25°C.

### Animal wound healing test:

The test animals are New Zealand minimal disease (MD)-grade white rabbits, aged 100 days, and weighed between 2.3 kg and 2.5 kg. Two days before the experiment, the rabbits were fitted with anti-licking back jackets for acclimation. The jackets may prevent the rabbits from licking the wounds but not interfere with their excretion, and may be easily removed for medicine administration and observation/photographing. On the day of starting the experiment, hair is removed from the central dorsal area of the rabbits. On both sides of the spine within the depilated area, 1 or 2 sites are selected per side as needed, and positions for creating 1.5 cm x 1.5 cm wounds (with the actual size as measured) are marked, and the wound edges are ensured to be 4 cm apart. Xylestesin-A is injected at the predetermined wound sites for anesthesia, then wounds are created using a biopsy punch and a scalpel, skin and flesh above the fascia are removed. Further, the wounds are pressed for hemostasis with medical gauze, and cleaned with saline solution, and finally dried with medical cotton balls, and then administered.

The administering dosage is 0.3 ml per wound for the first 7 days, and halved thereafter as the wound shrinked. After being administered, the wounds are covered and fixed with 3M breathable polyurethane film (TegadermTM Film, St. Paul, Minnesota). Administration is performed once every 24 hours, and the wound size is measured using the area measurement function of PHOTOSHOP software after being photographed. For the wounds in control group, all procedures are the same as the administered wounds except no pharmaceutical is applied.

### Comparison of Example 1 and Example 2:

Two pharmaceuticals: 3% Ge-CTS and 3% Ge-COS are used in Example 1; pharmaceutical of 3% Ge-Ca is used in Example 2.

The above three pharmaceuticals contain a Ge-132 equivalent concentration of 3%. For each pharmaceutical, two wounds are created on each side of the spine of three rabbits according to the above way, allowing three sets of experiments under identical conditions for the three pharmaceuticals and the untreated control group, and the wound areas are measured on days 1, 3, 7, and 14. Table 1 shows the experimental results of the two pharmaceuticals (3% Ge-132 and 3% Ge-COS) in Example 1, the pharmaceutical (3% Ge-Ca) in Example 2 , and the control group. The data contains wound area size and, for comparison, the proportional change in wound size based on the wound size on day 1 (i.e., 100%).

**Table 1: Experimental results of Example 1 and Example 2**

| Days of administration | | Day 1 | | Day 3 | | Day 7 | | Day 14 | |
|---|---|---|---|---|---|---|---|---|---|
| Wound area size | | cm² | Proportion | cm² | Proportion | cm² | Proportion | cm² | Proportion |
| Control group | Rabbit A | 2.12 | 100% | 2.85 | 114.3% | 1.34 | 63.3% | 0.26 | 12.2% |
| | Rabbit B | 2.30 | 100% | 2.20 | 95.7% | 1.22 | 53.0% | 0.26 | 11.3% |
| | Rabbit C | 2.41 | 100% | 2.77 | 110.0% | 1.76 | 72.9% | 0.12 | 5.0% |
| | Average | | 100% | | 106.7% | | 63.1% | | 9.5% |

| Example 1 | | | | | | | | | |
|---|---|---|---|---|---|---|---|---|---|
| 3% Ge-CTS | Rabbit A | 1.95 | 100% | 1.40 | 71.8% | 0.82 | 42.0% | 0.10 | 5.1% |
| | Rabbit B | 2.13 | 100% | 1.15 | 54.0% | 0.58 | 27.1% | 0.07 | 3.3% |
| | Rabbit C | 2.25 | 100% | 2.03 | 90.0% | 1.25 | 55.3% | 0.12 | 5.4% |
| | Average | | 100% | | 71.9% | | 41.5% | | 4.6% |
| 3% Ge-COS | Rabbit A | 2.32 | 100% | 1.57 | 67.7% | 0.95 | 40.9% | 0.08 | 3.4% |
| | Rabbit B | 2.21 | 100% | 1.06 | 48.0% | 0.64 | 29.1% | 0.11 | 5.0% |
| | Rabbit C | 2.25 | 100% | 2.23 | 99.0% | 1.47 | 65.2% | 0.13 | 5.8% |
| | Average | | 100% | | 71.5% | | 45.1% | | 4.8% |

| Example 2 | | | | | | | | | |
|---|---|---|---|---|---|---|---|---|---|
| 3% Ge-Ca | Rabbit A | 2.31 | 100% | 2.29 | 99.1% | 1.39 | 60.0% | 0.28 | 9.2% |
| | Rabbit B | 2.09 | 100% | 2.01 | 96.2% | 1.09 | 52.0% | 0.13 | 6.2% |
| | Rabbit C | 2.26 | 100% | 2.44 | 108.0% | 1.18 | 52.1% | 0.28 | 8.4% |
| | Average | | 100% | | 101.1% | | 54.7% | | 7.9% |

Generally, surgical wounds, whether administered or not, may tend to approach healing in about two weeks. When the pharmaceutical has the effect of promoting wound healing, the rate of wound contraction in the administered wounds is obviously faster than that of the untreated control group during the initial stage of the experiment. FIG. 2 shows the test results of each pharmaceutical in Example 1 for three rabbits on days 1, 3, 7, and 14. The two curves represent the proportional change in average wound size for the administered group and the control group, respectively; and the distance between the two curves represents the difference in wound contraction.

FIG. 2 shows that the wounds administered with either 3% Ge-CTS or 3% Ge-COS contract rapidly in the initial stage of administration and maintain a significantly faster healing rate than the control group throughout the experiment. The wound contraction rates of the administration of 3% Ge-CTS and 3% Ge-COS are very similar, with no significant difference. On the 3rd day, the wounds of the control group show obvious enlargement which is likely due to inflammation, whereas the administered wounds are free from this phenomenon. The experimental results of Example 1 fully demonstrate that Ge-CTS and Ge-COS have a highly significant effect of promoting wound healing, which may be attributed to the generation of TGFs and the pharmaceuticals' inhibition of inflammation.

### Example 2: wound healing experiment with Ge-132 alkaline-earth metal salts

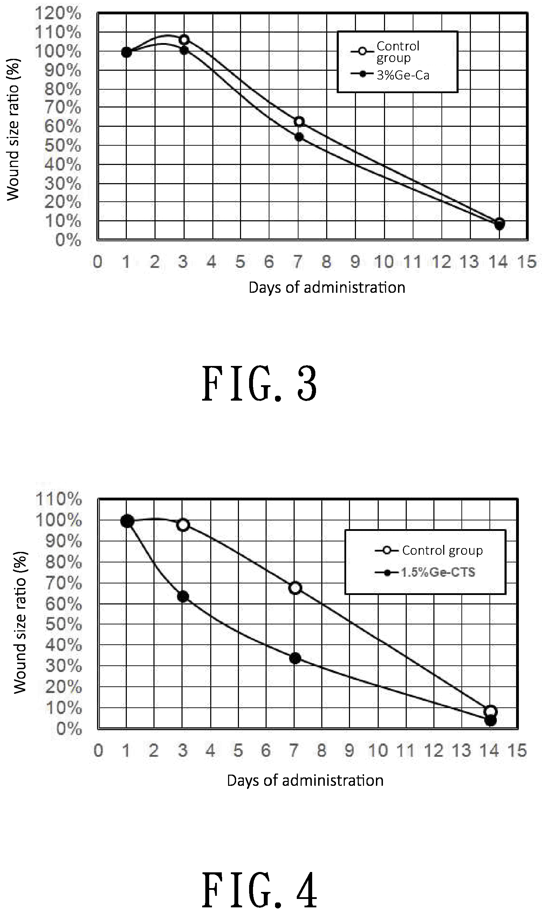

The proportional change in average wound size in Example 2 using 3% Ge-Ca is shown in FIG. 3. Both the wounds administered with 3% Ge-Ca and the wounds in control group show enlargement on the 3rd day, but the degree of enlargement in the administered group is significantly less than that in the untreated control group. The rate of wound contraction in the administered group is superior to that of the control group, and also better than that in the comparative example shown in FIG. 1 with 1.8% Ge-132 isotonic solution, but not as effective as the results in Example 1 with 3% Ge-CTS and 3% Ge-COS.

### Example 3: Healing effect experiment of low-concentration Ge-CTS

To understand the effect of pharmaceutical concentration in the present invention on wound healing, wound healing tests are particularly performed on three rabbits numbered D, E, and F using 1.5% Ge-CTS, separately. The wounds are created using the same method as in Example 1, with one 1.5 cm x 1.5 cm (with the actual size as measured) wound on each side of the spine of each rabbit. One wound is administered with 1.5% Ge-CTS, with the dosage as in Example 1, and the other wound is untreated as a control. The test results are shown in Table 2.

**Table 2: Experimental of Example 3**

| Days of administration | | Day 1 | | Day 3 | | Day 7 | | Day 14 | |
|---|---|---|---|---|---|---|---|---|---|
| Wound size | | cm² | Proportion | cm² | Proportion | cm² | Proportion | cm² | Proportion |
| Control group | Rabbit D | 2.43 | 100% | 2.32 | 95.5% | 1.61 | 66.4% | 0.27 | 11.2% |
| | Rabbit E | 2.53 | 100% | 2.66 | 105.3% | 1.92 | 75.7% | 0.19 | 7.5% |
| | Rabbit F | 2.23 | 100% | 2.09 | 93.5% | 1.37 | 61.6% | 0.16 | 7.1% |
| | Average | | 100% | | 98.1% | | 67.9% | | 8.6% |
| 1.5% Ge-CTS | Rabbit D | 2.03 | 100% | 1.12 | 55.0% | 0.61 | 30.2% | 0.06 | 2.9% |
| | Rabbit E | 2.34 | 100% | 1.74 | 74.4% | 0.87 | 37.0% | 0.10 | 4.1% |
| | Rabbit F | 2.42 | 100% | 1.50 | 62.2% | 0.85 | 35.0% | 0.13 | 5.2% |
| | Average proportion | | 100% | | 63.9% | | 34.1% | | 4.1% |

FIG. 4 shows the proportional change in average wound area on days 1, 3, 7, and 14 in Table 2 (with day 1 as 100%), indicating that wounds administered with 1.5% Ge-CTS contract much more obviously in the first 7 days than the untreated control group. Compared to the administration of 3% Ge-CTS in Example 1, the effect is equally remarkable and obviously superior to the effect of the Comparative Example 1 using 3% Ge-Ca, demonstrating that even at half concentration, Ge-CTS still has a significant effect of promoting wound healing and is markedly better than the Comparative Example 1 using 1.8% Ge-132 isotonic solution.

What is described above are merely preferred embodiments of the present invention but are not construed as limiting the present invention in any form. Although the present invention has been disclosed above in terms of preferred embodiments, it is not intended to limit the present invention. Those skilled in the art could make some changes or modifications to the disclosed methods and technical disclosure to produce equivalent embodiments without departing from the scope of the technical solution of the present invention. Moreover, any simple amendment, equivalent change, or modification made to the above embodiments within the technical solution of the present invention based on the technical essence of the present invention shall still fall within the scope of the technical solution of the present invention.

## Claims

1. A medicine, comprising a water-soluble salt compound that is prepared from an organogermanium compound carrying carboxylic acid group and a glucosamine compound carrying reactive amino group, via protonation of the reactive amino group of the glucosamine compound by a hydrogen ion on the carboxylic acid group of the organogermanium compound; wherein the glucosamine compound is selected from at least one of the following: glucosamine monosaccharide, chitooligosaccharide, and chitosan.

2. The medicine according to claim 1, wherein the medicine is a medicine having bioactivity and medical use derived from the organogermanium component and the glucosamine component contained therein.

3. The medicine according to claim 2, wherein the medical use derived from the organogermanium component is any one of the following: an anti-inflammatory action, an antiviral action, induction of interferon secretion, derivation of a transforming growth factor (TGF), and a wound healing effect.

4. The medicine according to claim 2, wherein the medical use derived from the glucosamine component is any one of the following: an antibacterial effect, an anti-inflammatory action, and a hemostatic effect.

5. The medicine according to claim 1, wherein the organogermanium compound carrying the carboxylic acid group is selected from at least one of the following: organogermanium sesquioxide, germane, germatrane, a spirogermanium compound, germanium porphyrine, and germanocene.

6. The medicine according to claim 1, wherein the chitooligosaccharide has a molecular weight of < 3,900 Da and a degree of deacetylation of >90%, and the chitosan has a molecular weight of <130 kDa and a degree of deacetylation of >90%.

7. The medicine according to claim 5, wherein the organogermanium sesquioxide has a monomer chemical formula as shown in Formula (II): 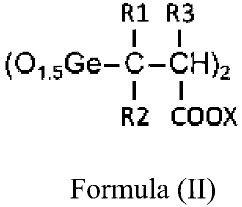 wherein:
R1, R2, and R3 in the formula is H, substituted or unsubstituted low-carbon alkyl, or substituted or unsubstituted amino group or amido group, wherein the substituted or unsubstituted low-carbon alkyl is methyl, ethyl, aryl, or hetaryl; and
X in the formula is H, K, Na, basic amino group, aryl, or hetaryl.

8. The medicine according to claim 7, wherein the organogermanium sesquioxide is bis(carboxyethyl)germanium sesquioxide (Ge-132).

9. The medicine according to claim 5, wherein the germatrane has a chemical formula as shown in Formula (IV): 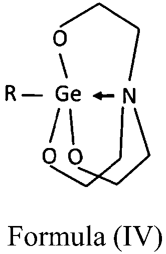 wherein:
R in the formula is cinnamic acid group [-CH(C₆H₄OH)CH₂COOH], caffeic acid group [-CH(C₆H₃(OH)₂)CH₂COOH], or any other groups comprising carboxylic acid group.

10. The medicine according to claim 5, wherein the spirogermanium compound is selected from at least one of the following: 3-(2,8,9-trioxa-aza-1-germatricyclo-[3,3,3,0]-undecane-1-yl) caffeic acid, 3-(2,8,9-trioxa-aza-1-germatricyclo-[3,3,3,0]-undecane-1-yl)-10-hydroxy-decanoic acid, 3-(2,8,9-trioxa-aza-1-germatricyclo-[3,3,3,0]-undecane-1-yl)-10-methoxy-decanoic acid, 3-(2,8,9-trioxa-aza-1-germatricyclo-[3,3,3,0]-undecane-1-yl)-10-ethoxy-decanoic acid, and 3-(2,8,9-trioxa-aza-1-germatricyclo-[3,3,3,0]-undecane-1-yl)-10-phenyl-decanoic acid.

11. The medicine according to claim 1, wherein the medicine is administered in a form selected from any one of the following: a solution, an emulsion, an ointment, a hydrogel, a powder, a film, and a nonwoven fabric.

12. The medicine according to claim 1, wherein the medicine further comprises an additive selected from at least one of the following: an antibiotic, a wetting agent, a vitamin, a thickener, an excipient, and a pH regulator.

13. The medicine according to claim 12, wherein the pH regulator is at least one of the following: acetic acid, formic acid, hydrochloric acid, sulfuric acid, ammonia water, sodium hydroxide, calcium hydroxide, and magnesium hydroxide.

14. A wound healing medicine, prepared by the following steps:
providing an organogermanium compound carrying carboxylic acid group;
providing a glucosamine compound, wherein the glucosamine compound carries active amino group capable of performing a protonation reaction, and is selected from at least one of the following: glucosamine monosaccharide, chitooligosaccharide, and chitosan;
mixing the organogermanium compound with the glucosamine compound in water, to generate protonation of the active amino group by the carboxylic acid group, and forming a pharmaceutical solution; and
adjusting a pH value and a weight of the pharmaceutical solution, and adding an excipient.

15. The wound healing medicine according to claim 14, wherein the organogermanium compound carrying the carboxylic acid group is selected from at least one of the following: organogermanium sesquioxide, germane, germatrane, a spirogermanium compound, germanium porphyrine, and germanocene.

16. The wound healing medicine according to claim 14, wherein the chitooligosaccharide has a molecular weight of < 3,900 Da and a degree of deacetylation of >90%, and the chitosan has a molecular weight of <130 kDa and a degree of deacetylation of >90%.

17. The wound healing medicine according to claim 15, wherein the organogermanium sesquioxide has a monomer chemical formula as shown in Formula (II): 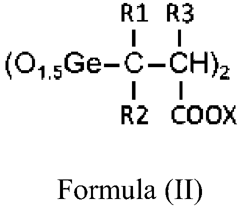 wherein:
R1, R2, and R3 in the formula is H, substituted or unsubstituted low-carbon alkyl, or substituted or unsubstituted amino group or amido group, wherein the substituted or unsubstituted low-carbon alkyl is methyl, ethyl, aryl, or hetaryl; and
X in the formula is H, K, Na, basic amino group, aryl, or hetaryl.

18. The wound healing medicine according to claim 17, wherein the organogermanium sesquioxide is bis(carboxyethyl)germanium sesquioxide (Ge-132).

19. The wound healing medicine according to claim 15, wherein the germatrane has a chemical formula as shown in Formula (IV): 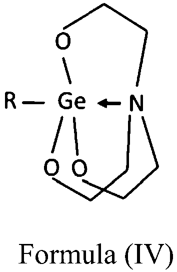 wherein:
R in the formula is cinnamic acid group [-CH(C₆H₄OH)CH₂COOH], caffeic acid group [-CH(C₆H₃(OH)₂)CH₂COOH], or any other groups comprising carboxylic acid group.

20. The wound healing medicine according to claim 15, wherein the spirogermanium compound is selected from at least one of the following: 3-(2,8,9-trioxa-aza-1-germatricyclo-[3,3,3,0]-undecane-1-yl) caffeic acid, 3-(2,8,9-trioxa-aza-1-germatricyclo-[3,3,3,0]-undecane-1-yl)-10-hydroxy-decanoic acid, 3-(2,8,9-trioxa-aza-1-germatricyclo-[3,3,3,0]-undecane-1-yl)-10-methoxy-decanoic acid, 3-(2,8,9-trioxa-aza-1-germatricyclo-[3,3,3,0]-undecane-1-yl)-10-ethoxy-decanoic acid, and 3-(2,8,9-trioxa-aza-1-germatricyclo-[3,3,3,0]-undecane-1-yl)-10-phenyl-decanoic acid, and 3-(2,8,9-trioxa-aza-1-germatricyclo-[3,3,3,0]undecane-1-yl)-hydroxycinnamic acid.

21. The wound healing medicine according to claim 14, wherein the step of adjusting the pH value of the pharmaceutical solution further comprises using a pH regulator selected from at least one of the following: acetic acid, formic acid, hydrochloric acid, ammonia water, sodium hydroxide, and magnesium hydroxide.

22. The wound healing medicine according to claim 14, wherein the medicine is for use in the treatment of any one of the following wounds: trauma, lacerations, abrasions, knife wounds, burns, scalds, sunburns, acnes, pressure sores, and diabetic ulcers.

23. The wound healing medicine according to claim 14, wherein the medicine is formulated for use in any one of the following forms: a solution, an emulsion, an ointment, a hydrogel, a powder, a film, and a nonwoven fabric.

24. A wound healing medicine, wherein the wound healing medicine comprises a salt formed by an organogermanium compound carrying carboxylic acid group and a compound selected from at least one of the following: glucosamine monosaccharide, chitooligosaccharide, chitosan, ammonium hydroxide, alkali metal hydroxide, and alkaline-earth metal hydroxide.

25. The wound healing medicine according to claim 24, wherein the alkali metal hydroxide is selected from at least one of the following: sodium hydroxide, potassium hydroxide, and lithium hydroxide; and the alkaline-earth metal hydroxide is selected from at least one of the following: calcium hydroxide, and magnesium hydroxide.

26. The wound healing medicine according to claim 24, wherein the organogermanium compound carrying the carboxylic acid group is selected from at least one of the following: organogermanium sesquioxide, germane, germatrane, a spirogermanium compound, germanium porphyrine, and germanocene.

27. The wound healing medicine according to claim 26, wherein the organogermanium sesquioxide is bis(carboxyethyl)germanium sesquioxide (Ge-132).

28. The wound healing medicine according to claim 26, wherein the germatrane has a chemical formula as shown in Formula (IV): 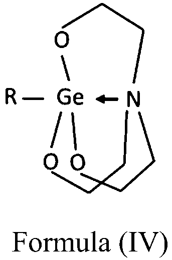 wherein:
R in the formula is cinnamic acid group [-CH(C₆H₄OH)CH₂COOH], caffeic acid group [-CH(C₆H₃(OH)₂)CH₂COOH], or any other groups comprising carboxylic acid group.

29. The wound healing medicine according to claim 26, wherein the spirogermanium compound is selected from at least one of the following: 3-(2,8,9-trioxa-aza-1-germatricyclo-[3,3,3,0]-undecane-1-yl) caffeic acid, 3-(2,8,9-trioxa-aza-1-germatricyclo-[3,3,3,0]-undecane-1-yl)-10-hydroxy-decanoic acid, 3-(2,8,9-trioxa-aza-1-germatricyclo-[3,3,3,0]-undecane-1-yl)-10-methoxy-decanoic acid, 3-(2,8,9-trioxa-aza-1-germatricyclo-[3,3,3,0]-undecane-1-yl)-10-ethoxy-decanoic acid, and 3-(2,8,9-trioxa-aza-1-germatricyclo-[3,3,3,0]-undecane-1-yl)-10-phenyl-decanoic acid.
